(19) Europäisches Patentamt
European Patent Office
Office européen des brevets

(11) Veröffentlichungsnummer: **0 168 749**
**B1**

(12) # EUROPÄISCHE PATENTSCHRIFT

(45) Veröffentlichungstag der Patentschrift: **27.04.88**

(21) Anmeldenummer: **85108466.5**

(22) Anmeldetag: **09.07.85**

(51) Int. Cl.⁴: **C 07 C 93/193**, C 08 F 20/34, C 07 C 101/18, C 07 C 143/18, C 07 F 9/38, C 07 C 121/407, C 07 C 69/74, C 07 F 9/54, C 07 C 13/15, C 07 C 103/70, C 07 C 143/34

(54) **Ionisch aufgebaute,polymerisierbare Verbindungen.**

(30) Priorität: **17.07.84 DE 3426197**

(43) Veröffentlichungstag der Anmeldung: **22.01.86 Patentblatt 86/4**

(45) Bekanntmachung des Hinweises auf die Patenterteilung: **27.04.88 Patentblatt 88/17**

(84) Benannte Vertragsstaaten: **BE DE FR GB**

(56) Entgegenhaltungen:
**FR-A-2 530 239**
**GB-A-758 992**
**US-A-2 677 699**
**US-A-3 131 227**
**US-A-3 936 492**
**US-A-4 105 858**
**US-A-4 138 441**
**US-A-4 176 232**

(73) Patentinhaber: **Agfa- Gevaert AG, Patentabteilung, D-5090 Leverkusen 1 (DE)**

(72) Erfinder: **Hoffarth, Gunther, Dr., Humboldtstrasse 25, D-5090 Leverkusen 3 (DE)**
Erfinder: **Richter, Wolfgang, Dr., Scheiblerstrasse 111, D-4150 Krefeld (DE)**
Erfinder: **Goossens, John, Ir., Roggendorfstrasse 51, D-5000 Köln 80 (DE)**
Erfinder: **Podszun, Wolfgang, Dr., Wolfskaul 4, D-5000 Köln 80 (DE)**
Erfinder: **Süling, Carlhans, Dr., Carl- Leverkus-Strasse 10, D-5068 Odenthal (DE)**
Erfinder: **Buysch, Hans- Josef, Dr., Brandenburger Strasse 28, D-4150 Krefeld (DE)**
Erfinder: **Walz, Klaus, Dr., Domblick 4, D-5090 Leverkusen 3 (DE)**

EP 0 168 749 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Gegenstand der vorliegenden Erfindung sind ionisch aufgebaute, polymerisierbare Verbindungen der allgemeinen Formel (I)

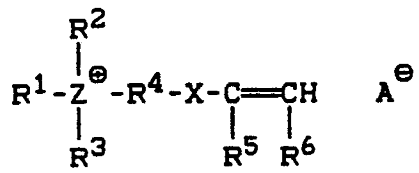 (I)

worin

Z für Stickstoff steht,

$R^1$, $R^2$ und $R^3$ gleich oder verschieden sind und Wasserstoff, geradkettige oder verzweigte Alkyl-, Alkenyl-, Aryl- oder Aralkylreste mit 1 bis 24 Kohlenstoffatomen bedeuten oder untereinander oder mit $R^4$ verknüpft sind und zusammen mit Z einen gesättigten oder ungesättigten Ring mit 3 bis 6 Kohlenstoffatomen bilden, wobei gegebenenfalls eine oder mehrere Kohlenstoffatome durch Heteroatome ersetzt sein können,

$R^4$ weiterhin für 1 bis 4 Methylengruppen steht,

$R^5$ und $R^6$ gleich oder verschieden sind und für Wasserstoff oder einen Alkylrest mit 1 bis 8 Kohlenstoffatomen stehen,

X:

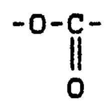 oder 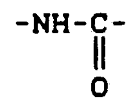

bedeutet und

$A^{\ominus}$ für ein Anion einer CH-aciden Verbindung mit mindestens einem Kohlenwasserstoffrest mit 6 bis 24 Kohlenstoffatomen steht.

Als Reste $R^1$, $R^2$ und $R^3$ der allgemeinen Formel (I) kommen in Frage geradkettige oder verzweigte Alkylreste mit 1 bis 24 Kohlenstoffatomen, bevorzugt 1 bis 18 Kohlenstoffatomen, wie der Methyl-, Ethyl-, Propyl-, Butyl-, Pentyl-, Hexyl-, Cyclohexyl-, Methylcyclohexyl-, Cyclopentyl-, Heptyl-, Octyl-, Decyl-, Dodecyl-, Tridecyl-, Stearyl- und Palmityl-Rest, bevorzugt der Methyl-, Ethyl-, Propyl-, Butyl-, Cyclohexyl-, Palmityl- und Stearyl-Rest; geradkettige oder verzweigte Alkenylreste mit 2 bis 24 Kohlenstoffatomen, bevorzugt 2 bis 4 Kohlenstoffatomen, wie der Vinyl- und Allyl-Rest, besonders bevorzugt der Vinylrest; Arylreste mit 6 bis 24 Kohlenstof fatomen, bevorzugt 6 bis 12 Kohlenstoffatomen, wie der Phenyl-, Naphthyl-, Methylphenyl- und Methylnaphthyl-Rest, bevorzugt der Phenylrest, oder Aralkylreste mit 7 bis 24 Kohlenstoffatomen, bevorzugt 7 bis 18 Kohlenstoffatomen, wie der Benzyl-, Phenethyl oder Naphthylmethyl-Rest, bevorzugt der Benzylrest.

Die Reste $R^1$, $R^2$ und $R^3$ in der allgemeinen Formel (I) können untereinander oder mit $R^4$ verknüpft sein und zusammen mit Z gesättigte oder ungesättigte Ringe mit 3 bis 6 Kohlenstoffatomen, bevorzugt 5 bis 6 Kohlenstoffatomen bilden, wobei gegebenenfalls ein- oder mehrere Kohlenstoffatome durch Heteroatome, wie Schwefel, Sauerstoff oder Stickstoff, bevorzugt Stickstoff, ersetzt sein können.

Als gesättigte oder ungesättigte ringformige Reste im Sinne der Erfindung seien genannt: der Azetidinyl-, Pyrrolidinyl-, Piperidinyl-, Azacycloheptanyl-, Pyrrolinyl-, Pyrrolyl-, 1H-Azepinyl-, Pyrazolyl-, Imidazolyl-, Oxazolyl-, Isoxazolyl-, Thiazolyl-, Imidazolinyl-, Imidazolidinyl-, Morpholinyl- und der Piperazinyl-Rest, bevorzugt der Pyrrolidinyl-, Imidazolinyl-, Imidazolidinyl- und der Piperazinyl-Rest.

Als Reste $R^5$ und $R^6$ in der allgemeinen Formel (I) kommen in Betracht Alkylreste mit 1 bis 8 Kohlenstoffatomen, bevorzugt 1 bis 4 Kohlenstoffatomen, wie der Methyl-, Ethyl-, Propyl-, Butyl-, Hexyl-, Cyclohexyl- und der Methylcyclohexyl-Rest, bevorzugt der Methyl- und der Ethyl-Rest.

Bevorzugt steht $R^4$ für 2 oder 3 Methylengruppen.

Das Anion $A^{\ominus}$ der CH-aciden Verbindung der Formel (I) kann in einer bevorzugt Ausführungsform folgender Formel entsprechen

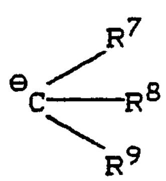 (II),

worin

$R^7$, $R^8$ und $R^9$ gleich oder verschieden sind und für CN, $COOR^{10}$, Halogen, $NO_2$ oder $SO_2R^{10}$ stehen, wobei $R^{10}$ einen Alkyl- oder Aralkylrest mit 1 bis 24 Kohlenstoffatomen darstellt mit der Maßgabe, daß mindestens einer

der Reste $R^7$ bis $R^9$ $COOR^{10}$ oder $SO_2R^{10}$ bedeutet und mindestens ein Rest $R^{10}$ 6 bis 24 Kohlenstoffatome besitzt, oder $R^7$ und $R^8$ zusammen mit dem zentralen Kohlenstoffatom einen Cyclopentadienylring der Formel (III)

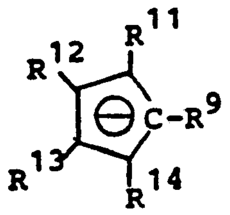

(III)

bilden,

in dem

$R^{11}$ bis $R^{14}$ gleich oder verschieden sind und für Wasserstoff, CN, $COOR^{10}$ oder einen Aklyl- oder Aralkylrest mit 6 bis 24 Kohlenstoffatomen stehen und

$R^9$ und $R^{10}$ die obengenannte Bedeutung haben mit der Maßgabe, daß mindestens einer der Reste $R^9$, $R^{11}$ bis $R^{14}$ $COOR^{10}$ oder einen Alkylrest mit 6 bis 24 Kohlenstoffatomen bedeuten und mindestens ein Rest $R^{10}$ 6 bis 24 Kohlenstoffatomen besitzt.

Als Reste $R^7$, $R^8$ und $R^9$ der allgemeinen Formel (II) kommen u.a. in Frage Halogene, wie Fluor, Chlor, Brom oder Iod, bevorzugt Fluor oder Chlor; die $COOR^{10}$-Gruppe; wobei $R^{10}$ ein Alkyl- oder Aralkylrest mit 1 bis 24 Kohlenstoffatomen, bevorzugt 1 bis 18 Kohlenstoffatomen, bedeutet und Methyl, Ethyl, Propyl, Butyl, Pentyl, Hexyl, Cyclohexyl, Methylcyclohexyl, Octyl, Decyl, Dodecyl, Tridecyl, Tetradecyl, Palmityl, Stearyl, bevorzugt Methyl, Ethyl, Propyl, Butyl, Pentyl, Hexyl, Decyl, Dodecyl, Tridecyl, Palmityl oder Stearyl sein kann, sowie die $SO_2R^{10}$-Gruppe mit der für $R^{10}$ angegebenen Bedeutung.

Als Reste $R^{11}$, $R^{12}$, $R^{13}$ und $R^{14}$ der allgemeinen Formel (III) seien u.a. genannt die $COOR^{10}$-Gruppe mit der zuvor genannten Bedeutung für $R^{10}$ sowie Alkyl- oder Aralkylreste mit 6 bis 24 Kohlenstoffatomen, bevorzugt 8 bis 18 Kohlenstoffatomen, wie der 2-Ethylhexyl-, Decyl-, Dodecyl-, Tridecyl-, Palmityl- und der Stearyl-Rest, bevorzugt der Decyl-, Dodecyl-, Tridecyl-, Palmityl- und der Stearyl-Rest.

Beispielhaft seien als Anionen einer CH-aciden Verbindung genannt:

Das Trisalkylsulfonylmethyl-anion, das Trisalkoxycarbonylmethyl-anion, das Pentaalkoxycarbonylcyclopentadienyl-anion, das Alkyl-tetraalkoxycarbonylcyclopentadienyl-anion, das Alkoxycarbonylhalogenmethyl-anion, das Alkoxycarbonylhalogennitromethyl-anion, das Tricyanalkylcyclopentadienyl-anion, das Tetracyanalkylcyclopentadienyl-anion und das Dicyanessigsäureestercarbanion.

Besonders bevorzugt sind Verbindungen mit Anionen der Formel

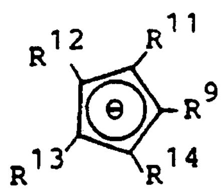

worin $R^9$ und $R^{11}$ bis $R^{14}$ gleich oder verschieden sind und für eine Alkylgruppe mit 6 - 24 C-Atomen steht.

Strukturell unterschiedliche ionisch aufgebaute polymerisierbare Verbindungen sind bereits bekannt geworden (US-A-2 677 699, US-A-4 105 858, US-A-4 176 232, GB-A-785 992, US-A-3 936 492 und FR-A1-2 530 239). Sie dienen zur Herstellung färbbarer Folien und Fasern, zur Herstellung von Ionenaustauscherharzen, als Zwischenprodukte zur Herstellung von Isocyanaten mit polymerisierbaren Gruppen, zur Herstellung von Textilhilfsmitteln, zur Herstellung selbststabilisierter Polymerdispersionen und zur Herstellung von Flockungsmitteln. Die Eignung der erfindungsgemäßen Verbindungen für die Herstellung elektrostatographischer Suspensionsentwickler ist aus diesem Stand der Technik nicht abzuleiten.

Bevorzugt sind die nachstehend formelmäßig aufgezählten neuen, ionisch aufgebauten, polymerisierbaren Verbindungen:

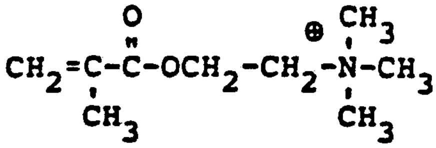 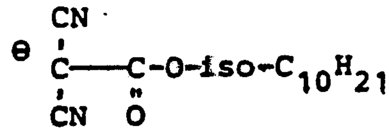

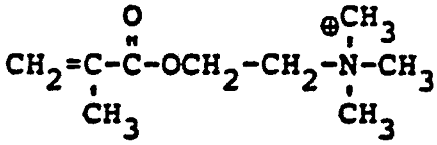 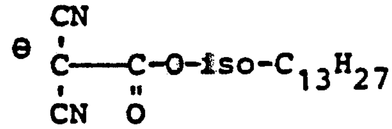

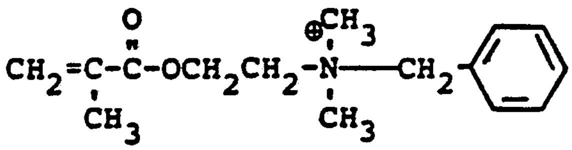 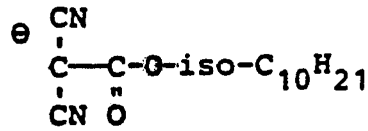

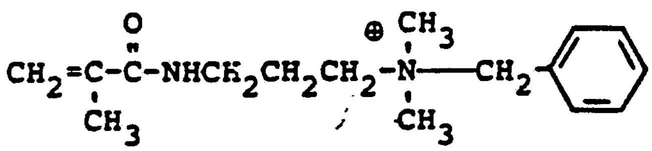 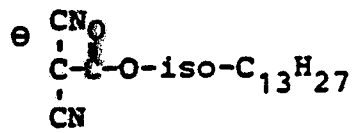

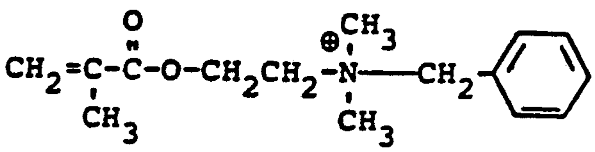 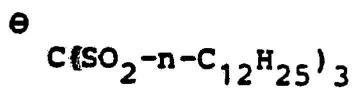

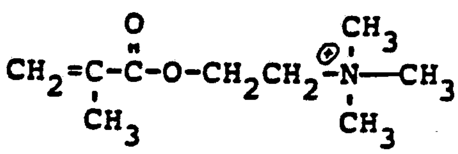 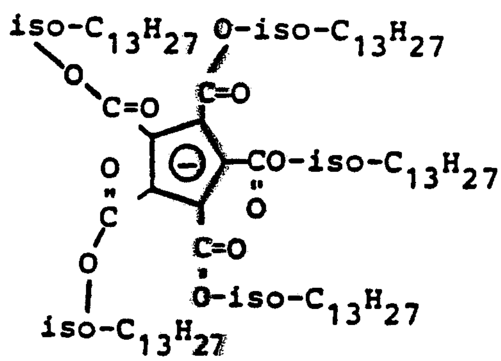

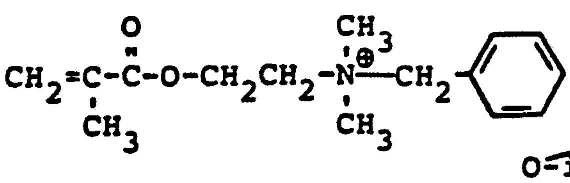 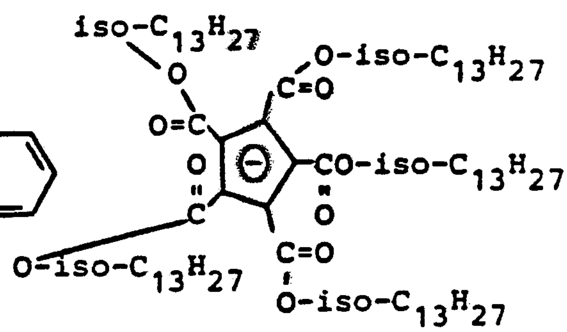

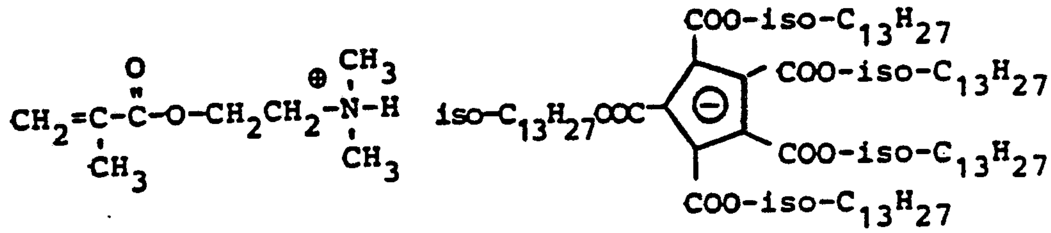

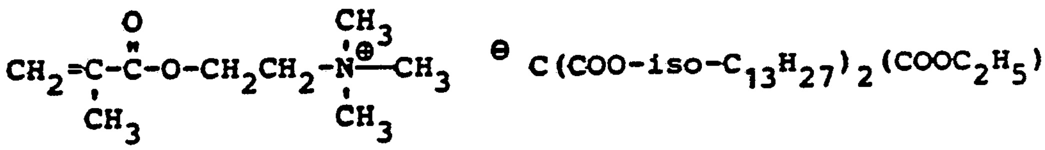

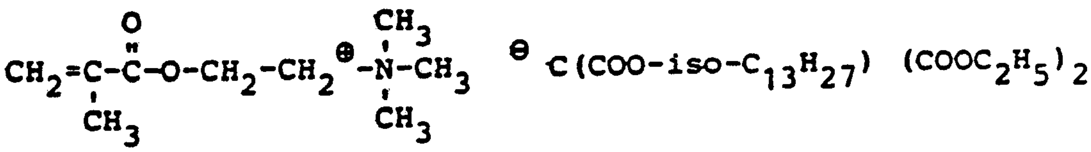

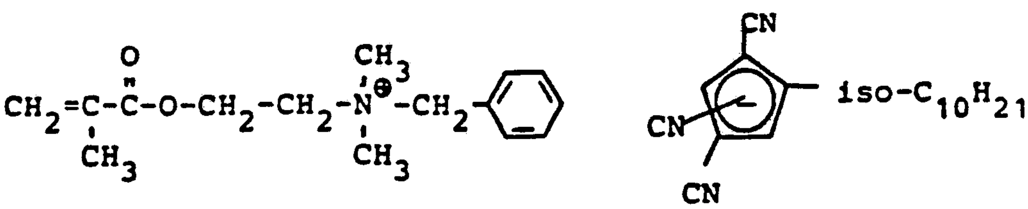

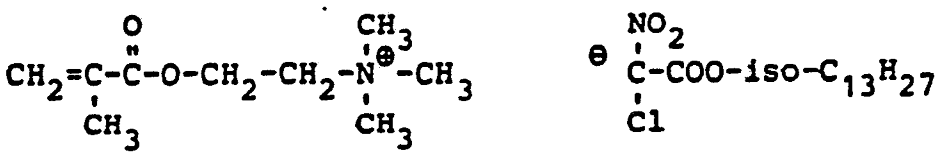

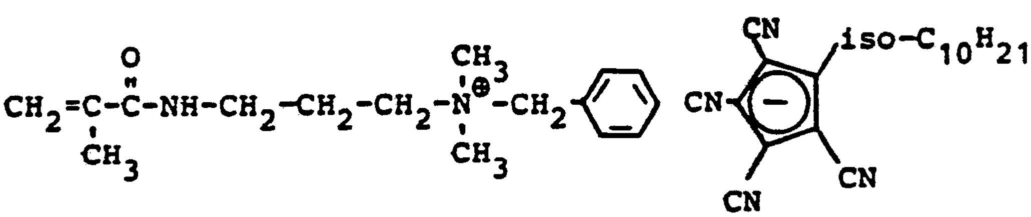

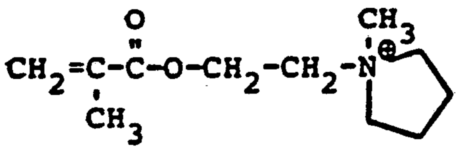
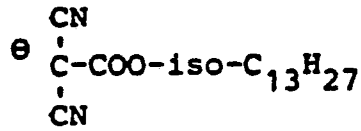

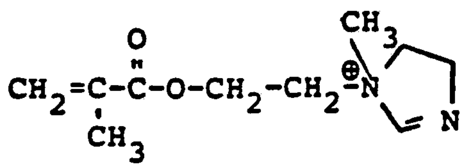
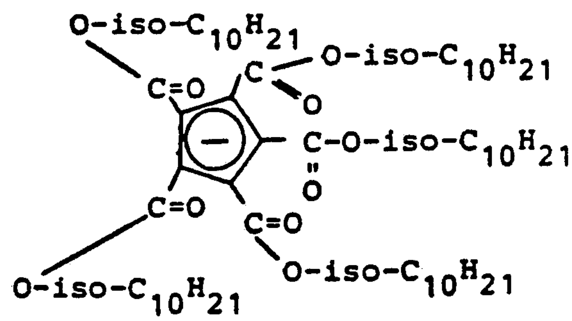

und

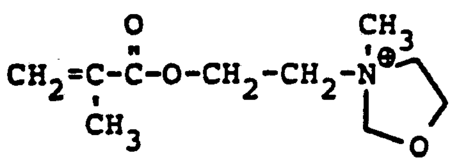
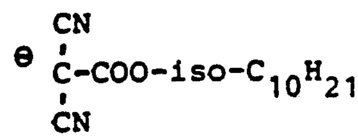
.

Besonders bevorzugt sind ionisch aufgebaute Verbindungen der Formeln:

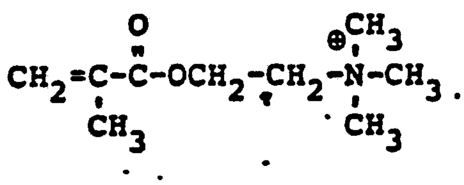
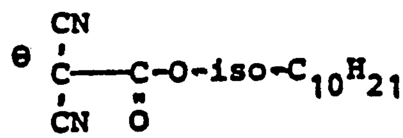

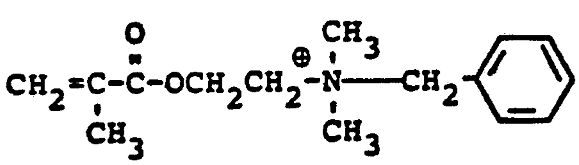
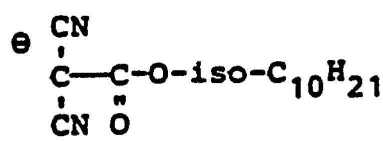

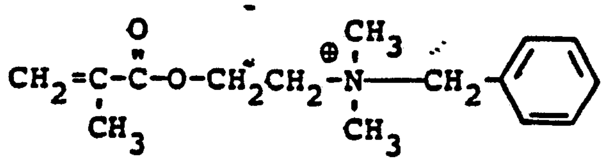

$^{\ominus}$ $C(SO_2-n-C_{12}H_{25})_3$

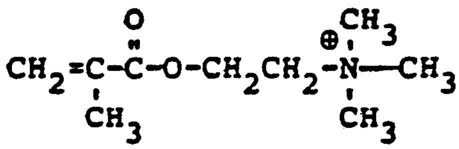

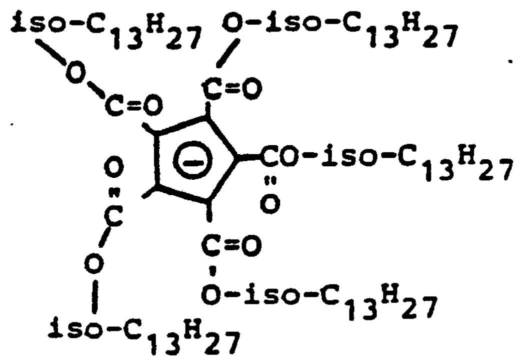

und

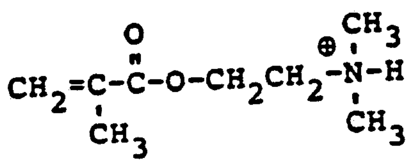

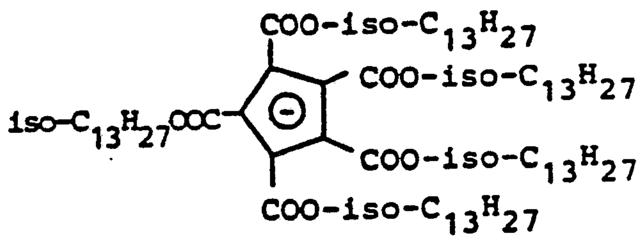

Die Verbindungen der Formel (I) werden durch Umsetzung der Verbindungen der Formel (IV)

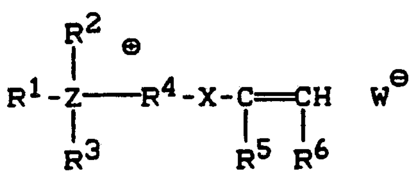

(IV),

worin

Z, $R^1$ bis $R^6$ und X die obengenannte Bedeutung besitzen und

W für Halogen, eine Alkylsulfat-, Alkyl- oder Arylsulfonat-, Alkylphosphit-, Alkylphosphonat- oder eine Dialkylphosphat-Gruppe steht,

mit Ammoniumsalzen oder Metallsalzen der Formel (V)

$M^{n\oplus} A_n^{\ominus}$ (V),

worin

An die obengenannte Bedeutung hat,

M für ein Metall der Gruppen Ia, IIa, Ib und IIb des Periodensystems der Elemente steht und

n 1 oder 2 bedeutet,

in wäßrigem und/oder polarem nichtwäßrigem Medium bei Temperaturen von etwa -20 bis 80°C, bevorzugt 0 bis 20°C, und anschließender Isolierung der ionischen Verbindungen durch Extraktion mit unpolaren Lösungsmitteln oder durch Einengen des Reaktionsgemisches hergestellt.

Als Halogene der Formel (IV) seien genannt: Fluor, Chlor, Brom oder Jod, bevorzugt Chlor oder Iod; als

Alkylsulfat-, Alkylsulfonat-, Alkylphosphit-, Alkylphosphonat- oder Dialkylphosphatreste solche mit 1 bis 4 Kohlenstoffatomen, bevorzugt 1 und 2 Kohlenstoffatomen, wie Methyl, Ethyl, Propyl oder Butyl, bevorzugt Methyl oder Ethyl. Als Metalle der Gruppen Ia, IIa, Ib und IIb des Periodensystems der Elemente (Mendelejew) kommen in Betracht: Lithium, Natrium, Magnesium, Silber und Zink, bevorzugt Lithium, Natrium und Kalium.

Die Umsetzung kann in Wasser und/oder nichtwäßrigem, polarem Medium durchgeführt werden. Als nichtwäßrige, polare Medien eignen sich Alkohole, Ether, Ketone, chlorierte Aliphaten, Nitrile und/oder Carbonsäureamide. Bevorzugt werden Alkohole, Ether und/oder Nitrile verwendet. Beispielsweise seien genannt: Methanol, Ethanol, Propanol, Isopropanol, Butanol, Isobutanol, Diethylether, Tetrahydrofuran, Dioxan, Aceton, Methylethylketon, Methylenchlorid, Chloroform, Acetonitril, Formamid, Methylformamid und/oder Dimethylformamid, bevorzugt Methanol, Ethanol, Tetrahydrofuran, Aceton, Chloroform, Methylenchlorid und/oder Acetonitril.

Üblicherweise erfolgt die Umsetzung der Verbindungen der Formel (IV) mit den Ammonium- oder Metallsalzen der Formel (V) in etwa stöchiometrischen Mengen. Beispielsweise können pro Äquivalent der Verbindungen der Formel (IV) 0,8 bis 1,2 Äquivalente, bevorzugt 0,9 bis 1,1 Äquivalente, an Ammonium- oder Metallsalzen der Verbindungen der Formel (V) eingesetzt werden.

Nach erfolgter Umsetzung lassen sich die neuen, ionisch aufgebauten, polymerisierbaren Verbindungen durch Extraktion aus dem Reaktionsgemisch mit unpolaren Lösungsmitteln, wie Aromaten, z.B. Benzol, Toluol und Xylol, chlorierte Aromaten, z.B. Chlorbenzol, Aliphaten, z. B. Petrolether, Hexan, Methylcyclohexan und Cyclohexan, und chlorierte Aliphaten, z. B. Chloroform, isolieren, sofern in wäßrigem Medium gearbeitet wurde. Wurde dagegen in polarem, nichtwäßrigem Medium gearbeitet, können die ionisch aufgebauten erfindungsgemäßen Verbindungen nach Abtrennen des Metallsalzes $M^{n\oplus}A_n^{\ominus}$ durch Abdestillieren des polaren Lösungesmittels isoliert werden.

Eine weitere Möglichkeit zur Herstellung der neuen, ionisch aufgebauten Verbindungen, welche konkreter die allgemeine Formel (VI) haben

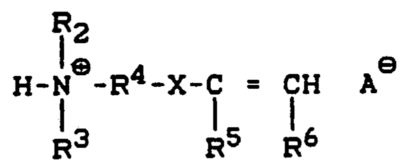

(VI),

worin

$R^2$ bis $R^6$ sowie X und A die zuvor genannte Bedeutung haben,

besteht darin, Verbindungen der allgemeinen Formel (VII)

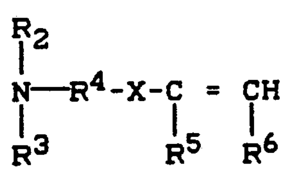

(VII),

worin

$R^2$ bis $R^6$ sowie X die zuvor genannte Bedeutung besitzen,

mit Verbindungen der allgemeinen Formel (VIII)

HA (VIII)

worin

A die bereits genannte Bedeutung hat,

vorzugsweise in Gegenwart von organischen Lösungs- und/oder Verdünnungsmitteln bei Temperaturen von -10 bis 40° C, bevorzugt 15 bis 25° C, umzusetzen.

Als organische Lösungs- und/oder Verdünnungsmittel können die bereits zuvor genannten organischen Lösungsund/oder Verdünnungsmittel eingesetzt werden.

Bei der Umsetzung, die eine Säure-Base-Reaktion darstellt, werden die Reaktionspartner wie üblich in stöchiometrischen Mengen eingesetzt.

Die Aufarbeitung erfolgt in üblicher Weise durch Abdestillieren des organischen Lösungs- und/oder Verdünnungsmittels, wobei die gewünschten Verbindungen quantitativ in reiner Form anfallen.

Die erfindungsgemäßen ionisch aufgebauten Verbindungen der Formel (I) sind sowohl zur Homopolymerisation als auch zur Copolymerisation befähigt. Derartige Polymere eignen sich z. B. für die Herstellung elektrostatographischer Suspensionsentwickler. In Hinblick auf ihre Verwendung als Bestandteil von Dispersionen ist jedoch die Copolymerisation von besonderem Interesse. Als Comonomere eignen sich bekannte Vinyl- und Vinylidenverbindungen. Beispielhaft seien aufgeführt: (Methyl)-Acrylsäure und ihre Derivate, wie (Meth)-Acrylsäureester, (Meth)-Acrylsäureamid, (Meth)-Acrylnitril, Vinylester, wie Vinylacetat, Vinylpropionat, ferner Diene, wie Butadien und Isopren, des weiteren Maleinsäureanhydrid, Maleinsäureimid und seine N-Alkyl- oder N-Arylimidderivate sowie halogenhaltige Monomere, wie Vinylchlorid und

Vinylidenchlorid. Gut geeignet sind auch Mischungen verschiedener Monomere. Gute Einbauarten werden vor allem dann erreicht, wenn zumindestens anteilmäßig (Meth)-Arylsäureester als Comonomere verwendet werden. Es können sowohl unvernetzte als auch durch Verwendung von mehr funktionellen Monomeren, wie Ethylendimethacrylat oder Divinylbenzol, vernetzte Copolymerisate aufgebaut werden.

Die Herstellung der Polymerisate kann nach an sich bekannten ionischen oder radikalischen Polymerisationsverfahren erfolgen, wie sie z. B. in Houben-Weyl, Methoden der Organischen Chemie, Bd. XIV, Makromolekulare Stoffe, Teil 1, 4. Auflage, Georg Thieme Verlag, Stuttgart 1961, beschrieben sind. Gute Ergebnisse werden bei radikalischen Polymerisationen in Substanz, in Lösung, in Emulsionen oder Suspensionen erzielt.

Die Einleitung der radikalischen Polymerisation kann mit üblichen Radikalbildnern, wie Peroxiden und vorzugsweise Azoverbindungen erfolgen. Auch eine Redoxpolymerisation, beispielsweise unter Verwendung eines Peroxid/Amin-Systems oder einer Photopolymerisation ist möglich.

Die nachfolgende Tabelle I im Beispielteil zeigt eine Reihe von Beispielen erfindungsgemäßer, ionisch aufgebauter Verbindungen. Zur Prüfung der Dissoziation der ionischen Verbindungen wurden die Äquivalentleitfähigkeiten bestimmt. Hierzu wurde die von Kohler (R. Kohler, D. Giglberger, F. Bestenreiner, Photographic Science and Engineering, 22, 2 (1978), 218-227) beschriebene Meßmethode angewendet. Tabelle II zeigt die Äquivalentleitfähigkeiten von bekannten und von erfindungsgemäßen Verbindungen, gemessen an 1 %-igen Lösungen in Toluol bei Raumtemperatur (Spalte A). Außerdem wurden die Äquivalentfähigkeiten bestimmt, nachdem die toluolischen Lösungen 24 Stunden bei Raumtemperatur in einer gesättigten Wasserdampfatmosphäre gelagert waren (Spalte B). Tabelle III zeigt entsprechende Ergebnisse für Lösungen in Isododecan. Die Ergebnisse lassen erkennen, daß die Leitfähigkeit der erfindungsgemäßen Verbindungen in der Regel hoher sind als die der bekannten Verbindungen.

Die Tabelle II und III zeigen darüberhinaus deutlich, daß die Leitfähigkeiten der erfindungsgemäßen Verbindungen weit weniger feuchtigkeitsempfindlich sind als die der bekannten Verbindungen (B/A-Verhältnis). Aus den für die Polymere 1-6 (Tabelle III) ermittelten Werten geht hervor, daß die gute Leitfähigkeit und geringe Feuchtigkeitsempfindlichkeit auch für Copolymerisate gilt, die die erfindungsgemäßen ionischen Verbindungen einpolymerisiert enthalten.

Die gegenüber den bekannten Verbindungen 7-9 erhöhte Leitfähigkeit der erfindungsgemäßen Substanzen sowie der daraus hergestellten Polymere 1-6 in unpolaren Lösungsmitteln wird durch einen größeren Dissoziationsgrad hervorgerufen. Zwangsläufig damit gekoppelt ist eine erhöhte Ladungsdichte auf der Oberfläche der dispergierten Teilchen, welches zu verminderter Agglomeration und damit zu verbesserter Stabilität von Dispersionen mit den erfindungsgemäßen Substanzen führt.

Eine verbesserte Lagerstabilität laßt sich auch aus der deutlich geringeren Feuchteempfindlichkeit der erfindungsgemäßen Substanzen und ihrer Polymere verglichen mit Verbindungen 7-9 ablesen.

**Tabelle I**

Erfindungsgemäße ionische Verbindungen

1 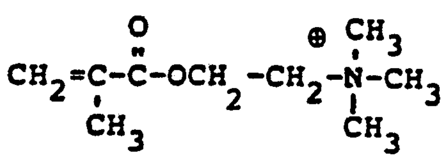 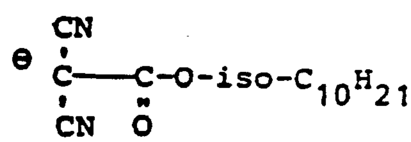

2 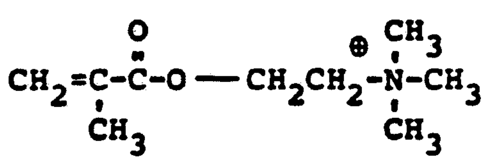 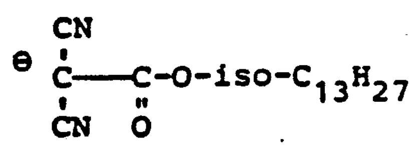

3 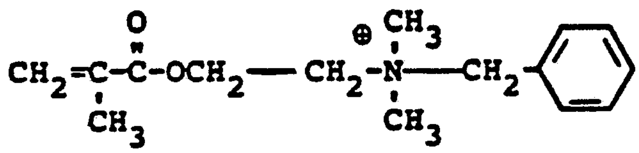 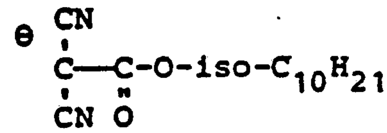

4 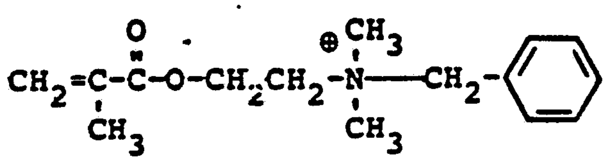 $^{\ominus}$ $C(SO_2-n-C_{12}H_{25})_3$

5 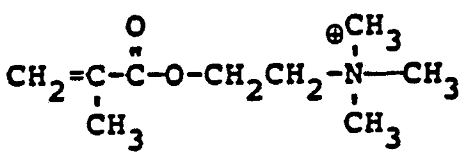 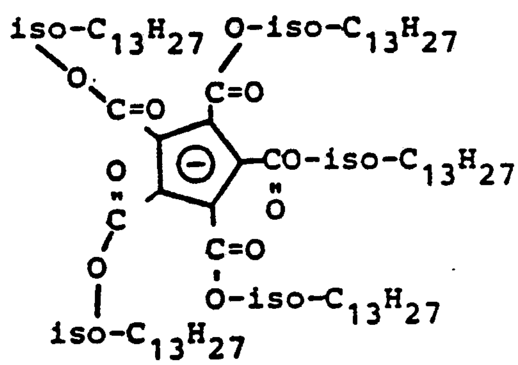

6 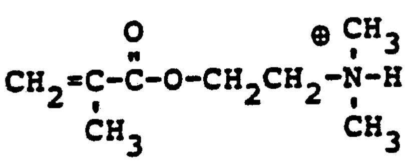 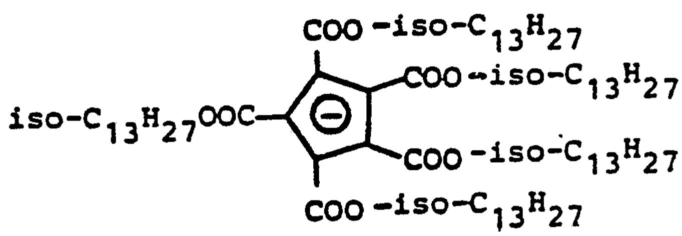

Copolymerisate einiger erfindungsgemäßer ionischer Verbindungen

| | |
|---|---|
| Pol 1 | 10 Gew.-% Verbindung 3<br>45 Gew.-% Styrol<br>45 Gew.-% Laurylmethacrylat |
| Pol 2 | 10 Gew.-% Verbindung 4<br>45 Gew.-% Styrol,<br>45 Gew.-% Laurylmethacrylat |
| Pol 3 | 10 Gew.-% Verbindung 6<br>45 Gew.-% Styrol<br>45 Gew.-% Laurylmethacrylat |
| Pol 4 | 10 Gew.-% Verbindung 1<br>90 Gew.-% Laurylmethacrylat |
| Pol 5 | 10 Gew.-% Verbindung 1<br>70 Gew.-% Laurylmethacrylat<br>20 Gew.-% Divinylbenzol |
| Pol 6 | 10 Gew.-% Verbindung 6<br>90 Gew.-% Laurylmethacrylat |

Bekannte, nicht polymerisierbare, ionische Verbindungen:

| Verbindung Nr. | |
|---|---|
| 7 | Natriumdi(2-ethylhexyl)sulfosuccinat |
| 8 | Zn-di(2-ethylhexyl)phosphat |
| 9 | Zn-hexyldecylsulfonat |

**Tabelle II**

Äquivalentleitfähigkeiten [$\Omega^{-1}m^{-1}kg\ äq^{-1}$] gemässen in Tuluol bei Raumtemperatur

| Verbindung Nr. | Wert A (trocken) | Wert B (nach Lagerung über $H_2O$) | B/A |
|---|---|---|---|
| 1 er- | $1,6 \cdot 10^{-5}$ | $2,6 \cdot 10^{-6}$ | 0,2 |
| 3 fin- | $4,0 \cdot 10^{-5}$ | $4,0 \cdot 10^{-5}$ | 1,0 |
| 4 dungs- | $2,0 \cdot 10^{-5}$ | $2,2 \cdot 10^{-5}$ | 1,1 |
| 6 gemäß | $5,5 \cdot 10^{-7}$ | $5,1 \cdot 10^{-7}$ | 9,0 |
| 7 | $5,6 \cdot 10^{-7}$ | $4,9 \cdot 10^{-6}$ | 8,8 |
| 8 | $6,0 \cdot 10^{-6}$ | $2,1 \cdot 10^{-5}$ | 3,5 |
| 9 | $4,9 \cdot 10^{-7}$ | $9,5 \cdot 10^{-6}$ | 19,4 |

**Tabelle III**

Äquivalentleitfähigkeiten [$\Omega^{-1}m^{-1}kg\ äq^{-1}$] gemessen in Isododecan bei Raumtemperatur

| Verbindung Nr. | Wert A (trocken) | Wert B (nach Lagerung über $H_2O$ | B/A |
|---|---|---|---|
| Pol 5 | $3{,}5 \cdot 10^{-6}$ | $2{,}8 \cdot 10^{-6}$ | 0,8 |
| Pol 6 | $1{,}1 \cdot 10^{-7}$ | $1{,}7 \cdot 10^{-7}$ | 1,55 |
| 4 | $3{,}0 \cdot 10^{-5}$ | $1{,}3 \cdot 10^{-5}$ | 0,43 |
| 7 | $9{,}2 \cdot 10^{-8}$ | $1{,}0 \cdot 10^{-5}$ | 109 |
| 8 | $2{,}0 \cdot 10^{-5}$ | $0{,}3 \cdot 10^{-7}$ | 0,04 |
| 9 | $2{,}1 \cdot 10^{-8}$ | $1{,}8 \cdot 10^{-5}$ | 857 |

**Beispiel 1**

Zu 15,0 g (170 mmol) Natriummalodinitril in 150 ml abs. Ethanol werden 19,5 g (88,3 mmol) Chlorameisensäureisodecylester in 20 ml Diethylether bei -10° C innerhalb von 30 Min. getropft. Dann läßt man auf Raumtemperatur kommen und erwärmt anschließend 30 Min. zum Rückfluß (68° C). Der Niederschlag von 5,7 g NaCl (Theorie 5,1 g) wird abfiltriert und das Filtrat zur Trockene eingeengt. Der Rückstand (26,8 g) wird mit Methylenchlorid versetzt, um Malodinitril herauszulosen, dann abgesaugt und mehrfach mit Methylenchlorid gewaschen. Der Niederschlag wird im Ölpumpenvakuum getrocknet:
19,5 g (81 % d. Th.) Na-dicyanessigsäureisodecylester

Elementaranalyse:

| | | | | |
|---|---|---|---|---|
| gef.: | C 60,9 % | H 7,69 % | N 10,8 % | Na 8,1 % |
| ber.: | 61,7 | 7,77 | 10,3 | 8,4 |

**Beispiel 2**

Analog werden aus 15,5 g (176 mmol) Natriummalodinitril, 150 ml abs. Ethanol und 37,7 g (88,2 mmol) Chlorameisensäureisotridecylester 17,1 g (62 % d. Th.) Na-dicyanessigsäureisotridecylester erhalten.

Elementaranalyse:

| | | | | |
|---|---|---|---|---|
| gef.: | C 63,9 % | H 8,78 % | N 9,25 % | Na 7,1 % |
| ber.: | 64,9 | 8,66 | 8,9 | 7,3 |

**Beispiel 3** (Verbindung 1 aus Tabelle I)

Zu 4,1 g (15 mmol) Na-dicyanessigsäureeisodecylester, gelöst in 20 ml Wasser, werden bei Raumtemperatur 4,7 g (15 mmol) 2-Methacryloxyethyl-trimethylammoniumiodid in 20 ml Wasser gegeben. Augenblicklich fällt ein braungrünes Öl aus, welches mit Methylenchlorid extrahiert wird. Die organische Phase wird über Natriumsulfat getrocknet, filtriert und Methylenchlorid mit $N_2$ bei Raumtemperatur abgedampft. Die Ausbeute an 2-Methacryloxyethyl-trimethylammonium-dicyanessigsäureisodecylester ist quantitativ.

Elementaranalyse:

| | | | |
|---|---|---|---|
| gef.: | C 64,6 % | H 9,11 % | N 10,4 % |
| ber.: | 65,53 | 9,32 | 10,0 |

**Beispiel 4** (Verbindung 2 aus Tab. I)

Zu 4,7 g (15 mmol) Na-dicyanessigsäureisotridecylester, gelöst in 20 ml Wasser, werden bei Raumtemperatur 4,7 g (15 mmol) 2-Methacryloxyethyl-trimethylammoniumiodid in 20 ml Wasser gegeben. Augenblicklich fällt ein braungrünes Öl aus, welches man mit Methylenchlorid extrahiert. Die organische Phase wird über Natriumsulfat getrocknet, filtriert und Methylenchlorid mit $N_2$ abgedampft.

Die Ausbeute an 2-Methacryloxyethyl-trimethylammonium-dicyanessigsäureisotridecylester beträgt 6,6 g (90 %. d. Th.).

| | | | |
|---|---|---|---|
| gef.: | C 65,6 | H 9,73 | H 9,5 |
| ber.: | 66,48 | 10,04 | 9,3 |

**Beispiel 5** (Verbindung 3 aus Tab. I)

4,1 g (15 mmol) Na-dicyanessigsäureisodecylester, gelöst in 20 ml Wasser, werden bei Raumtemperatur mit 4,95 g (15 mmol) Benzyl-2-methacryloxyethyl-dimethylammonium-chlorid in 20 ml Wasser versetzt. Sofort fällt ein braun-grünes Öl aus, welches man mit Methylenchlorid extrahiert. Die organische Phase wird über Natriumsulfat getrocknet, filtriert und Methylenchlorid mit Stickstoff abgedampft. Die Ausbeute an Benzyl-2-methacryloxyethyldimethylammonium-dicyaneessigsäureisodecyleser beträgt 5,9 g (70 %. d. Th.).

| | | | |
|---|---|---|---|
| gef.: | C 69,1 | H 8,54 | N 8,5 |
| ber.: | 70,0 | 8,71 | 8,4 |

**Beispiel 6**

Zu 12,3 g (20 mmol) Trithioorthoameisensäure-tri-n-dodecylester in 150 ml 1,2-Dichlorpropan werden bei ca. 10° C innerhalb von 2 h 30 g (180 mmol) 46 %-ige Peressigsäure getropft. Anschließend läßt man den Ansatz auf Raumtemperatur kommen und 3 h nachreagieren. Dann wird 30 Min. auf 82° C erwärmt, 250 ml Wasser zugefügt und im Wasserstrahlvakuum 1,2-Dichlorpropan abdestilliert. Der ausgefallene Feststoff wird abgesaugt, mit Wasser gewaschen und luftgetrocknet: 9,1 g (feucht) aus 90 ml Ethanol umkristallisiert, 6,26 g (44 % d. Th.) Tris-(n-dodecylsulfonyl)-methan. Schmelzpunkt 126,5 bis 128,5° C.

Elementaranalyse:

| | | | | |
|---|---|---|---|---|
| gef.: | C 62,6 % | H 10,9 %. | S 13,3 % | O 13,8 % |
| ber.: | 62,3 | 10,7 | 13,5 | 13,5 |

**Beispiel 7** (Verbindung 4 aus Tab. I)

5,21 (7,04 mmol) Tris-(n-dodecylsulfonyl)-methylnatrium werden in abs. Methanol/THF gelöst. Dazu fügt man bei Raumtemperatur 1,97 g (7,04 mmol) Benzyl-2-methacryloxyethyl-dimethylammoniumchlorid. Der in geringen Mengen entstehende Niederschlag wird abfiltriert und das Filtrat zur Trockne eingeengt. Es werden 5,72 g (84,6 % d. Th.) Benzyl-2-methacryloxyethyl-dimethylammonium-tris-(n-dodecylsulfonyl)-methyl in Form eines farblosen Feststoffs vom Schmelzpunkt 65,5 bis 69° C erhalten.

| | | | | |
|---|---|---|---|---|
| gef.: | C 63,8 | H 9,88 | N 1,3 | S 9,6 |
| ber.: | 65,0 | 10,18 | 1,46 | 10,0 |

**Beispiel 8**

7,13 g (20 mmol) 1,2,3,4,5-Pentamethoxycarbonylcyclopentadien werden mit 20,0 g (100 mmol) Isotridecylalkohol versetzt und bei 100° C innerhalb von 8 h zunächst bei 1013 mbar, anschließend bei 363 bis 18 mbar 3,12 g (97,5 % d. Th.) Methanol abdestilliert. Rückstand 24 g 1,2,3,4,5-Pentaisotridecyloxycarbonylcyclopentadien.

**Beispiel 9** (Verbindung 5 aus Tab. 1)

4,94 g (4,2 mmol) 1,2,3,4,5-Pentaisotridecyloxycarbonylcyclopentadien werden in 30 ml Ethanol gelöst und mit 0,5 g (5 mmol) Kaliumacetat versetzt. Nachdem eine homogene Lösung enstanden ist, wird Ethanol und entstandene Essigsäure im Ölpumpenvakuum abdestilliert. Der verbleibende ölige Rückstand wird in wenig Ethanol gelöst und mit 1,3 g (4,1 mmol) 2-Methacryloxyethyl-trimethyl-ammoniumiodid in 10 ml 80 %-igem Ethanol versetzt. Nach Abdestillieren des Lösungsmittels im Vakuum wird der Rückstand in Methylenchlorid aufgenommen, filtriert, das Filtrat zur Trockne eingeengt, nochmals in Methylenchlorid aufgenommen und filtriert. Nach Abdampfen des Lösungsmittels - zuletzt mit $N_2$ - wer den 5,5 g (98,8 % d. Th.) 2-Methacryloxyethyl-trimethylamminum-1,2,3,4,5-pentaisotridecyloxycarbonylcyclopentadienid in Form eines oliv-braunen Öls erhalten.

| | | | |
|---|---|---|---|
| gef.: | C 72,8 | H 10,9 | N 1,1 |
| ber.: | 73,69 | 11,26 | 1,0 |

**Beispiel 10** (Verbindung 6 aus Tab. I)

4,79 g (4 mmol) 1,2,3,4,5-Pentaisotridecyloxycarbonylcyclopentadien werden mit 0,66 g (4 mmol) Dimethylaminoethylmethacrylat innigst vermischt.
In exothermer Reaktion entsteht 2-Methacryloxyethyl-dimethylammonium-1,2,3,4,5-pentaisotridecyloxycarbonylcyclopentadienid als grünes Öl; 5,45 g (quantitativ).

| | | | |
|---|---|---|---|
| gef.: | C 73,4 | H 11,1 | N 1,0 |
| ber.: | 73,57 | 11,23 | 1,0 |

**Beispiel 11** (Pol 1-3)

2 g ionische Substanz Nr. 3, 4 oder 6 (aus Tab. I) 9 g Styrol, 9 g Laurylmethacrylat und 20 mg Azodiisobuttersäuredinitril werden in 20 g Toluol gelöst und 6 Stunden bei 80°C gerührt. Das gebildete Polymerisat wird durch Ausfällen mit Methanol isoliert, durch Umfällen gereinigt und elementaranalytisch untersucht.

| Pol Nr. | ionische Substanz Nr. (Tab I) | Umsatz | Polymerisat (berechnet) N | S | (gefunden) N | S |
|---|---|---|---|---|---|---|
| 1 | 3 | 75% | 0,84 | - | 0,90 | - |
| 2 | 4 | 85% | 0,15 | 1,00 | 0,15 | 0,90 |
| 3 | 6 | 70% | 0,10 | - | 0,15 | - |

**Beispiel 12** (Pol 4)

Entsprechend Beispiel 11 werden 2 g ionische Substanz 1 aus Tab. I und 18 g Laurylmethacrylat copolymerisiert. Man erhält ein Copolymerisat mit 75 % Ausbeute.

**Beispiel 13** (Pol 5)

2 g ionische Substanz 1 aus Tab. I, 14 g Laurylmethacrylat, 4 g Divinylbenzol und 60 mg Azodiisobuttersäuredinitril werden in 80 g Toluol gelöst und 12 Stunden unter Rühren auf 80°C erhitzt. Man erhält eine stabile Dispersion mit 17,5 % Polymerisatanteil.

**Beispiel 14** (Pol 6)

Entsprechend Beispiel 12 werden 2 g ionische Substanz Nr. 6 aus Tab. I und 18 g Laurylmethacrylat copolymerisiert.

## Patentansprüche

1. Ionisch aufgebaute, polymerisierbare Verbindungen der Formel

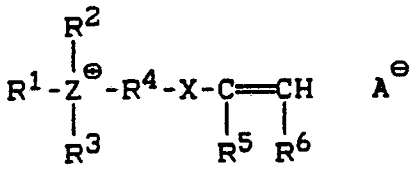
(I)

worin

Z für Stickstoff steht,

$R^1$, $R^2$2 und $R^3$ gleich oder verschieden sind und Wasserstoff, geradkettige oder verzweigte Alkyl-, Alkenyl-, Aryl- oder Aralkylreste mit 1 bis 24 Kohlenstoffatomen bedeuten oder untereinander oder mit $R^4$ verknüpft sind und zusammen mit Z einen gesättigten oder ungesättigten Ring mit 3 bis 6 Kohlenstoffatomen bilden, wobei gegebenenfalls ein oder mehrere Kohlenstoffatome durch Heteroatome ersetzt sein können,

$R^4$ weiterhin für 1 bis 4 Methylengruppen steht,

$R^5$ und $R^6$ gleich oder verschieden sind und für Wasserstoff oder einen Alkylrest mit 1 bis 8 Kohlenstoffatomen stehen,

X: 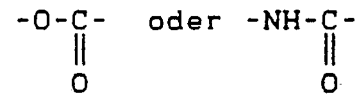

bedeutet und

$A^\ominus$ für ein Anion CH-aciden Verbindung mit mindestens einem Kohlenwasserstoffrest mit 6 bis 24 Kohlenstoffatomen steht.

2. Polymerisierbare Verbindung nach Anspruch 1, dadurch gekennzeichnet, daß $A^\ominus$ folgende Struktur besitzt,

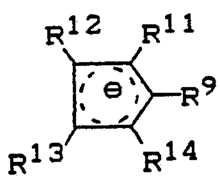

worin $R^9$ und $R^{11}$ bis $R^{14}$ gleich oder verschieden sind und für Alkyl mit 6-24 C-Atomen stehen.

## Claims

1. Ionically structured polymerisable compounds of the formula

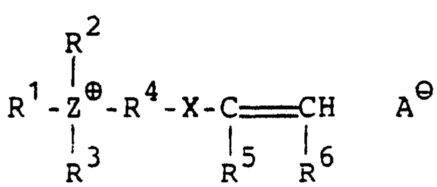
(I)

wherein

Z stands for nitrogen,

$R^1$, $R^2$ and $R^3$ may be identical or different and denote hydrogen or straight chained or branched alkyl, alkenyl, aryl or aralkyl groups with 1 to 24 carbon atoms or together with one another or linked to $R^4$ and together with Z they may form a saturated or unsaturated ring having 3 to 6 carbon atoms, one or more carbon atoms being optionally replaced by hetero atoms,

$R^4$ may also stand for 1 to 4 methylene groups,

$R^5$ and $R^6$ may be identical or different and denote hydrogen or an alkyl group with 1 to 8 carbon atoms,

X denotes

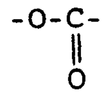 or 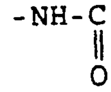

and

$A^{\ominus}$ stands for an anion of a CH-acidic compound containing at least one hydrocarbon group having 6 to 24 carbon atoms.

2. Polymerisable compound according to claim 1, characterised in that $A^{\ominus}$ has the following structure

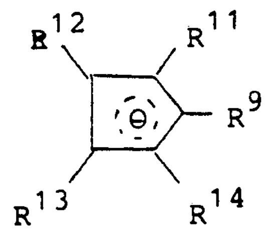

wherein $R^9$ and $R^{11}$ to $R^{14}$ are identical or different and stand for alkyl with 6 to 24 carbon atoms.

## Revendications

1. Composés polymérisables, à structure ionique, de formule

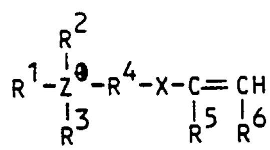 $A^{\ominus}$ (I)

dans laquelle

Z représente l'azote,

$R^1$, $R^2$ et $R^3$, ayant des significations identiques ou différentes, représentent l'hydrogène, des groupes alkyle à chaîne droite ou ramifiée, alcényle, aryle ou aralkyle contenant 1 à 24 atomes de carbone, ou bien ils sont reliés entre eux ou avec $R^4$ et forment alors ensemble et avec Z un cycle saturé ou insaturé contenant 3 à 6 atomes de carbone et dans lequel le cas échéant un ou plusieurs atomes de carbone peuvent être remplacés par des hétéroatomes,

$R^4$ peut en outre représenter de 1 à 4 groupes méthylene,

$R^5$ et $R^6$, ayant des significations identiques ou différentes, représentent chacun l'hydrogène ou un groupe alkyle en $C_1$-$C_8$,

X représente

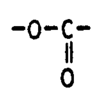 ou 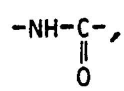,

et

$A^{\ominus}$ représente un anion d'un composé à CH acide ayant au moins un radical hydrocarboné en $C_6$-$C_{24}$.

2. Composé polymérisable selon la revendication 1, caractérisé en ce que $A^{\ominus}$ possède la structure suivante

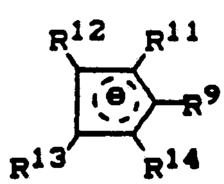

dans laquelle $R^9$ et $R^{11}$ à $R^{14}$, ayant des significations identiques ou différentes, représentant chacun un groupe alkyle en $C_6$-$C_{24}$.